# EUROPEAN PATENT SPECIFICATION

(11) **EP 4 398 825 B1**
(45) Date of publication and mention of the grant of the patent: **03.12.2025**
(21) Application number: 22776917.1
(22) Date of filing: 07.09.2022
(51) Int. Cl.: A61B 17/80

(54) **APPARATUS FOR CANINE ELBOW CORRECTION**
VORRICHTUNG ZUR KORREKTUR DES KNIEBOGENS EINES HUNDES
APPAREIL DE CORRECTION DE COUDE CANIN

(30) Priority: 10.09.2021 US 202163242665 P
(43) Date of publication of application: 17.07.2024
(73) Proprietor: Kyon AG, 8005 Zürich (CH)
(72) Inventor: PFEIL, Ingo, 01465 Dresden (DE); BRESINA, Stephen, 7260 Davos (CH)
(74) Representative: Weickmann & Weickmann PartmbB
(86) International application number: PCT/EP2022/074820
(87) International publication number: WO 2023/036803

(56) References cited:
- FR-A1- 2 294 685
- US-A1- 2008 045 960
- US-A1- 2008 103 595
- US-A1- 2012 265 254
- US-A1- 2016 278 828
- US-A1- 2018 325 568

## Description

### TECHNICAL FIELD

The present invention relates to an apparatus for correcting misalignments of the paw in the foreleg of dogs and other animals. The realignment of the paw is intended to properly align the medial compartment of the elbow with the end of the humerus.

### BACKGROUND OF THE INVENTION

Arthritis of the elbow joint is the most common cause of foreleg lameness in dogs. Most of the arthritic diseases of the elbow are considered forms of dysplasia. Common causes of dysplasia include fragmented coronoid process (FCP) and medial compartment disease (MCD). In FCP, the most common form of elbow dysplasia in dogs, a fragment of bone and/or cartilage of the ulna is broken off. The fragment may move and cause additional damage to the joint. Furthermore, the broken bone changes the relationship between abutting surfaces of the humerus and ulna. This changed relationship causes a misalignment of the forces affecting the joint, particularly when loaded. The misaligned forces can cause cartilage damage and/or further damage to the bones.

Treatment for FCP requires removal of any bone fragments. However, this may not be sufficient if the joint has been otherwise damaged. If the joint has been severely damaged or the bones are misaligned, further damage to the joint and ongoing lameness are likely.

One method for correcting problems with the elbow joint is to replace all or part of the joint. However, elbow replacement requires extensive and complicated surgery. Despite the existence of various elbow prostheses, none has proven safe and effective for routine use.

Sliding humeral osteotomy (SHO) has been proposed by Dr. Schultz at the Orthopedic Research Laboratory of the University of California, Davis. The procedure realigns the humerus to shift the forces off an area of cartilage damage. In this procedure, the humerus is cut above the elbow. A plate is used to reposition portions of the humerus bone.

Proximal abducting ulnar (PAUL) osteotomy developed by Dr. Pfeil, Dresden, Germany, and described in WO 2010/121233 shifts the distal forelimb outwards (lateral) and readjusts the loading through the elbow to a more lateral position, thus decreasing load being placed onto the surface of the ulna and medial condyle of the femur and shifting the load to the lateral side of the joint..

There is a bi-oblique proximal osteotomy of the ulna, which is good for allowing the distribution of the forces between the medial and lateral sides of the elbow joint to balance out. This method is useful in dogs from about 6 months to 12 months of age. A distal ulnar ostectomy can be performed to achieve the same outcome as the bi-oblique method, but is only applicable to much younger dogs (4 to 6 months of age).

A distal abducting ulnar osteotomy (DAUL) readjusts the loading through the elbow to a more lateral position, thus decreasing load being placed onto the surface of the ulna and medial condyle of the femur and shifting the load to the lateral side of the joint.

US 2008/103595 is directed to a bone plating system, and in particular to a system for the fixation of a canine humerus following a slide osteotomy.

### DISCLOSURE OF THE INVENTION

The present invention provides a device for realignment of a paw of a mammal, particularly a dog, to the sagittal plane by adjusting the torsion of the ulna. The torsion of the ulna is corrected by cutting it into two parts, preferably near the distal end of the bone. A plate is provided for attachment to the ulna to adjust the torsion of the ulna. The plate has a proximal and a distal part. One part is straight, and the other part is angled around the long axis of the plate. Different plates may have different angles of this twist angle. The plate with the desired angle of twist is selected to achieve the correct torsion of the ulna to bring the paw into alignment with the sagittal plane. The twist of the plate is either clockwise or counter-clockwise for left or right side applications. The plate is preferably adapted for attachment to the lateral-caudal side of the ulna. The cut is easiest performed towards the distal end, but may be performed anywhere between the distal to the proximal end, provided there is sufficient space for the plate to sit on the bone without encroaching the joint. Associated methods are also described herein to aid understanding of the invention, but these do not form part of the claimed invention.

A not claimed procedure for realigning the humerus and ulna bones in the elbow of a dog or another mammal is disclosed to correct for the changed geometry of the head of the ulna from a damage to the elbow join such as FCP. The ulna is cut above the wrist joint. The torsion of the lower part of the ulna is adjusted so that the outward angle of the paw is adjusted towards normal of between 5 and 10 degrees. Often times the damage at the elbow joint causes the outward angle of the paw to reach in excess of 60 degrees. Once the paw is rotated to the correct position, the torsion of the ulnar is fixed in this position with a special plate and screw implant to hold the 2 parts of the ulna together at the new angle.

According to another aspect of the invention, the torsion of the paw is measured when the forelimb is held in an extended position and the wrist is hyperextended. The measurement of the outward torsion angle of the paw in this position is used to determine the error in alignment.

The necessary change in the torsion angle of lower portion of the ulna is determined based upon the misalignment measured by the increase of the outward direction of the paw. Once the cut is made to the distal ulna, the paw is rotated into a normal position of between 0 and 10 degrees of outward torsion.

A plate is provided to reconnect the portions of the ulna where it is cut. The plate has 2 flat portions that are twisted relative to one another. A flat portion of the plate is adapted for attachment to the upper portion of the ulna. Another a flat portion of the plate.is adapted for attachment to the lower portion of the ulna. The upper and lower flat portions of the plate are twisted about the long axis of the plate. The plate is adapted for attachment to the parts of the ulna by fixing means, typically bone screws. According to another aspect of the invention, plates are provided with different degrees of twist angles and for right and left sides. The plate used depends upon the desired adjustment in the twist angle required to fix the plate to the lateral surface of the ulna upper and lower to the cut.

The apparatus of the present invention are particularly useful in veterinary medicine, more particularly in the treatment of dog.

### DESCRIPTION OF THE DRAWINGS

Figure 1: A view of a normal canine forelimb skeleton and the sagittal plane. The mechanical axis of the elbow joint is shown as the line, which projects laterally from the lateral humeral epicondyle and medially from the medial humeral epicondyle. The sagittal plane is perpendicular to this axis. In this instance, the axis of the paw is lined up with the sagittal plane.
Figure 2: A view of a canine forelimb skeleton and the sagittal plane. In this view, the axis of the paw is not congruent with the sagittal plane and is externally rotated by the angle β.
Figure 3: A perspective view of a plate with holes for accepting screws. The underside of the plate has contact to the bone only at points at each side of the screw holes. There is an angular twist to the plate between the distal and proximal segments of the plate. The plate is used to connect the distal and proximal portions of an osteotomy of the ulnar and to induce a twist between the two bone segments.
Figure 4: A side view of the plate used to fix the distal portion of the ulna in a rotated orientation.
Figure 5: The end view of the plate. There will be a range of plates with different twist angles. Plates also need to be produced mirrored for Right and Left sides. The angle alpha can be as small as 5 degrees and potentially as large as 50 degrees.
Figure 6: A view of the ulna with the plate applied towards the distal end. The plate is placed on the caudal-lateral side of the ulna. The distal component is now rotated inwards to correct the torsion of the ulna.

### DETAILED DESCRIPTION OF PREFERRED EMBODIMENTS

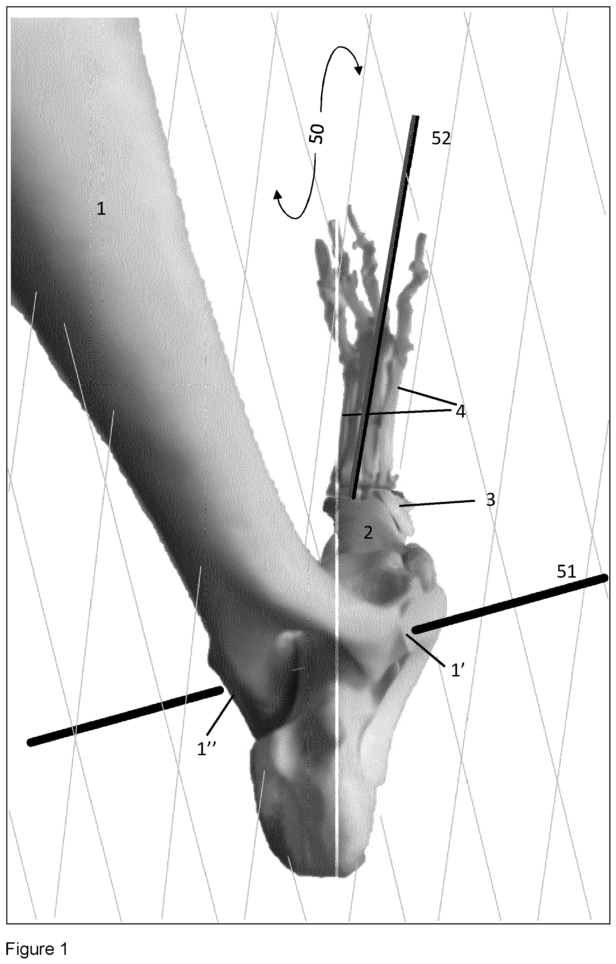

Fig. 1 shows the normal situation of the bones involved with the elbow joint, the humerus 1, the radius 2 and the ulna 3. The mechanical axis of the joint 52 is often viewed as the vector passing through the medial epicondyle 1" of the humerus through the lateral epicondyle of the humerus 1'. The sagittal plane 50 is perpendicular to this axis at a level of the center of the elbow joint. The paw, which is made up of a group of carpal bones, a set of longitudinal extending metacarpals 4 and phalanges will line up 51 with the sagittal plane or be rotated slightly to the lateral direction by 5 to 10 degrees in a normal elbow when the paw is in extension.

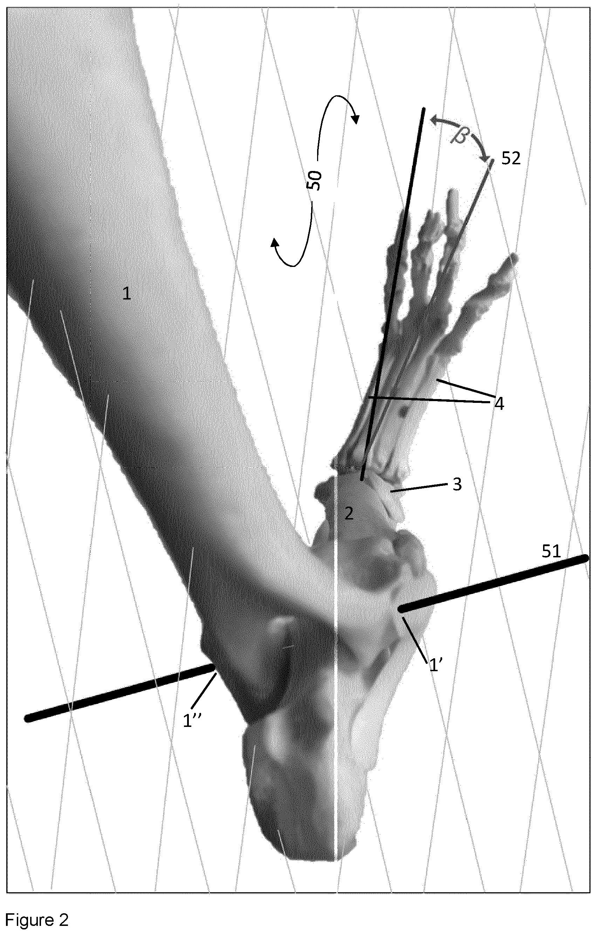

When the joint is loaded, i.e. when the dog is standing, the humerus is supported evenly across the head of the radius 2 and the ulna 3. However, with elbow dysplasia, the bones do not interact evenly, and the axis of the paw 51 is externally rotated, as shown in Fig. 2, by an angle β much larger than the normal 5 to 10 degrees.

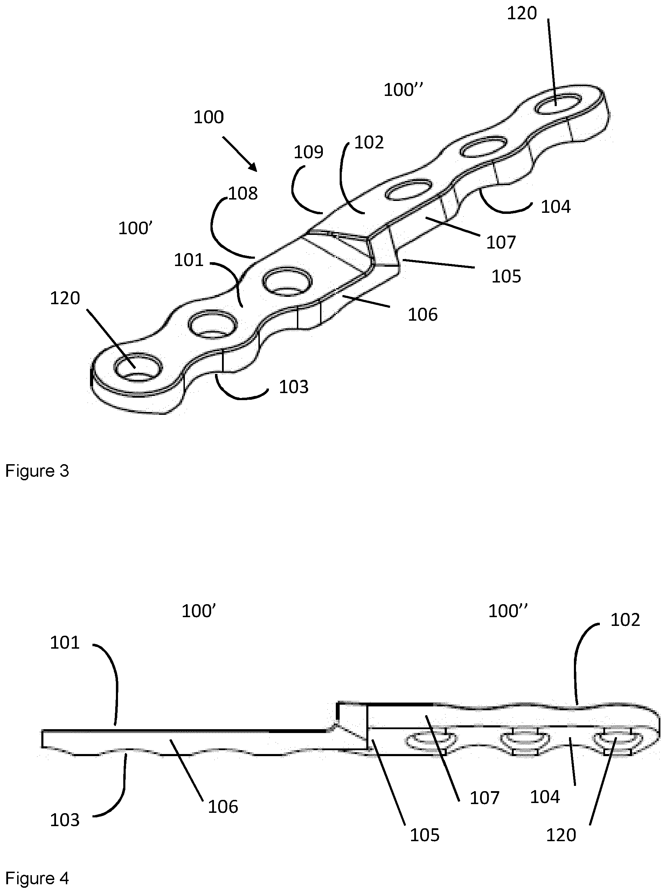

Figs. 3, 4 and 5 illustrate a plate 100 for use in correcting a misalignment of the paw by readjusting the torsion of the ulna. The plate 100 is elongated with a proximal part 100' and a distal part 100". It is similar to other types of plates for attaching to bones. It may be of any material, but is preferably a metal. The surface may be formed or treated to allow bone in-growth or on-growth. The plate 100 has first side 101 and a second side 103 opposite the first side 101 in the proximal part 100' and a first side 102 and a second side 104 opposite the first side 102 in the distal part 100". The first sides 101 and 102 are preferably flat. The second sides 103 and 104 have preferably a non-planar surface in order to limit the contact to the bone. More preferably, the second sides 103 and 104 are formed to provide contact with the bone only around screw holes 120.The other sides 106, 107, 108, and 109 may also be planar, or may have various other shapes. As is known with bone plates, the sides 106, 107, 108, and 109 may be curved about screw holes 120. The plate 100 has a plurality of screw holes 120 for receiving bone screws to attach the plate to a bone. Preferably, the screw holes 120 are recessed so that the heads of the screws are positioned below the first sides 101 and 102 of the plate. Of course, other types of screw holes could be used. Any type of bone screw, including locking and non-locking screws, can be used to attach the plate 100 to the ulna. Different types of screws could be used in different holes. The plate 100 in Figs. 3 and 4 has been illustrated with six screw holes 120. However, any number of screw holes, e.g. 4, 6 or 8 could be used.

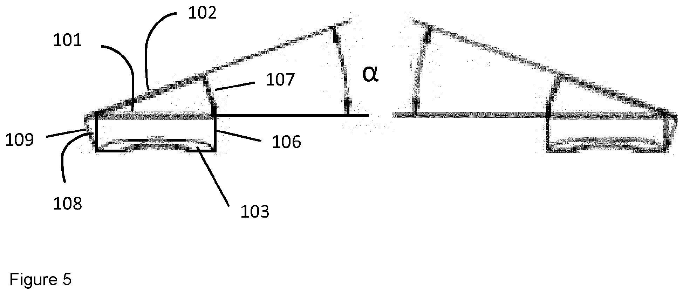

In Fig.5 it is illustrated how the distal part of the plate 100" is oriented by an angle α relative to the proximal part of the plate 100'. The two parts of the plate 100' and 100" are joined by the angled portion 105. The angled portion 105 may be substantially perpendicular to the first surface 101. The angled portion 105 may also be substantially perpendicular to the second surface 104. At the angled portion 105, there is a triangular step between first surfaces 101 and 102. There is a similar shaped step between second surfaces 103 and 104. In the proximal part of the plate 100', the second side 103 is substantially parallel to the first side 101. In the distal part of the plate 100", the second surface 104 is substantially parallel to the first surface 102.

In order to correct the misalignment of the paw and to further correct the torsion of the ulna, a measurement of the angle β is made using a goniometer. Experimental and clinical data will provide guidance for how much angular twist of the plate α is required for the measured angle β.

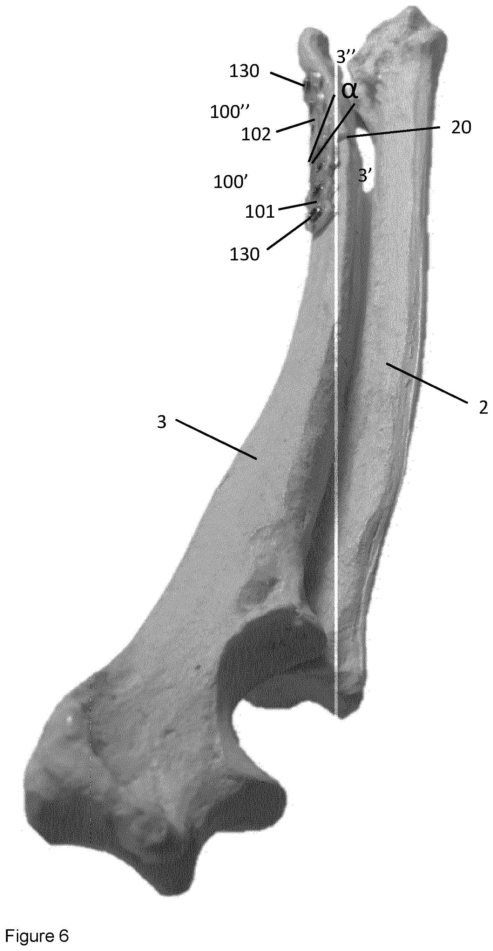

As illustrated in Fig. 6, the ulna 3 is cut at position 20 into proximal and distal parts 3' and 3". Preferably, the cut is straight across and perpendicular to the ulna 3. However, other types of cuts could be used. Preferably, the cut is not close to the distal end of the ulna. The cut must be far enough away from the end of the distal radius 2 such that the plate does not interfere with the joint. The plate 100 is attached to the parts 3' and 3" of the ulna in the ordinary manner. Screws 130 are positioned into the holes 120. The plate may be clamped to the parts of the bone before attachment with the screws to maintain the desired positions. The plate is attached to a flattened area of the ulna on the lateral-caudal aspect of the ulna.

## Claims

1. A plate (100) for adjusting the angle of the medial compartment of an ulna by adjusting the torsion of the ulna, wherein the plate (100) comprises:
a first portion (100') and a second portion (100");
the first portion (100') including a first side (101), and a second side (103) spaced apart from and parallel to the first side (101);
the second portion (100") including a third side (102), and a fourth side (104) spaced apart from and parallel to the third side (102);
an angled portion bridge (105) connecting first portion (100') to second portion (100"), such that first (100') and second (100") portion are aligned in the long axis of the plate (100), wherein the angled portion (105) is perpendicular to the first side (101) and the fourth side (104);
an angle (α) between the first surface (101) of the first portion (100') and the third surface (102) of the second portion, which is between about 5 degree and about 60 degree; and
a plurality of screw holes (102) formed in the plate (100) from the first side (101) to the third side (102) in the first portion (100') and from the second side (103) to the fourth side (104) of the second portion (100") to attach the plate (100) to bone.

2. The plate (100) of claim 1, which is adapted for attachment to the ulna after cutting the ulna near its distal end.

3. The plate (100) of claim 2, wherein the first portion (100') is adapted for attachment to the proximal part of the cut ulna and the second portion (100") is adapted for attachment to the distal part of the cut ulna.

4. The plate of any one of claims 1-3, wherein the angle between the first surface of the first portion and the third surface of the second portion is selected based on the error in alignment determined by measuring the paw torsion when the forelimb is held in an extended position and the wrist is hyperextended.

5. The plate (100) of any one of claims 1-4, wherein the plate (100) is adapted for attachment to the lateral-caudal side of the ulna.

6. The plate (100) of any one of claims 1-5, which is adapted for adjusting the angle of the medial compartment of an ulna in a dog.

## Patentansprüche

1. Eine Platte (100) zum Einstellen des Winkels des medialen Kompartiments einer Ulna durch Einstellen der Torsion der Ulna, wobei die Platte (100) umfasst:
einen ersten Abschnitt (100') und einen zweiten Abschnitt (100");
wobei der erste Abschnitt (100') eine erste Seite (101) und eine zweite Seite (103), die von der ersten Seite (101) beabstandet und parallel zu dieser ist, umfasst;
wobei der zweite Abschnitt (100") eine dritte Seite (102) und eine vierte Seite (104), die von der dritten Seite (102) beabstandet und parallel zu dieser ist, umfasst;
einen abgewinkelten Brückenabschnitt (105), der den ersten Abschnitt (100') mit dem zweiten Abschnitt (100") verbindet, sodass der erste (100') und der zweite (100") Abschnitt entlang der Längsachse der Platte (100) ausgerichtet sind, wobei der abgewinkelte Abschnitt (105) senkrecht zu der ersten Seite (101) und der vierten Seite (104) ist;
einen Winkel (α) zwischen der ersten Oberfläche (101) des ersten Abschnitts (100') und der dritten Oberfläche (102) des zweiten Abschnitts, der zwischen etwa 5 Grad und etwa 60 Grad beträgt; und
eine Vielzahl von Schraubenlöchern (102), die in der Platte (100) von der ersten Seite (101) zur dritten Seite (102) im ersten Abschnitt (100') und von der zweiten Seite (103) zur vierten Seite (104) des zweiten Abschnitts (100") ausgebildet sind, um die Platte (100) am Knochen zu befestigen.

2. Die Platte (100) nach Anspruch 1, die zur Befestigung an der Ulna ausgebildet ist, nachdem die Ulna in der Nähe ihres distalen Endes durchtrennt worden ist.

3. Die Platte (100) nach Anspruch 2, wobei der erste Abschnitt (100') zur Befestigung am proximalen Teil der durchtrennten Ulna ausgebildet ist und der zweite Abschnitt (100") zur Befestigung am distalen Teil der durchtrennten Ulna ausgebildet ist.

4. Die Platte nach einem der Ansprüche 1-3, wobei der Winkel zwischen der ersten Oberfläche des ersten Abschnitts und der dritten Oberfläche des zweiten Abschnitts basierend auf dem Ausrichtungsfehlers ausgewählt ist, der durch Messen der Pfotentorsion ermittelt ist, wenn das Vorderbein in einer gestreckten Position gehalten wird und das Vorderfußwurzelgelenk überstreckt ist.

5. Die Platte (100) nach einem der Ansprüche 1-4, wobei die Platte (100) zur Befestigung an der lateral-kaudalen Seite der Ulna ausgebildet ist.

6. Die Platte nach einem der Ansprüche 1-5, die zum Einstellen des Winkels des medialen Kompartiments einer Ulna bei einem Hund ausgebildet ist.

## Revendications

1. Plaque (100) pour ajuster l'angle du compartiment médial d'un cubitus en ajustant la torsion du cubitus, la plaque (100) comprenant :
une première partie (100') et une deuxième partie (100") ;
la première partie (100') présentant une première face (101) et une deuxième face (103) espacée de la première face (101) et parallèle à celle-ci ;
la deuxième partie (100") présentant une troisième face (102) et une quatrième face (104) espacée de la troisième face (102) et parallèle à celle-ci ;
une partie angulaire formant pont (105) reliant la première partie (100') à la deuxième partie (100"), de telle sorte que les première (100') et deuxième (100") parties soient alignées dans l'axe longitudinal de la plaque (100), la partie angulaire (105) étant perpendiculaire à la première face (101) et à la quatrième face (104) ;
un angle (α) entre la première surface (101) de la première partie (100') et la troisième surface (102) de la deuxième partie, qui est compris entre environ 5 degrés et environ 60 degrés ; et
une pluralité de trous taraudés (120) formés dans la plaque (100), allant de la première face (101) à la troisième face (102) dans la première partie (100') et de la deuxième face (103) à la quatrième face (104) dans la deuxième partie (100"), pour fixer la plaque (100) à l'os.

2. Plaque (100) selon la revendication 1,
adaptée pour être fixée au cubitus après sectionnement du cubitus près de son extrémité distale.

3. Plaque (100) selon la revendication 2,
dans laquelle la première partie (100') est adaptée pour être fixée à la partie proximale du cubitus sectionné, et la deuxième partie (100") est adaptée pour être fixée à la partie distale du cubitus sectionné.

4. Plaque selon l'une des revendications 1 à 3,
dans laquelle l'angle entre la première surface de la première partie et la troisième surface de la deuxième partie est sélectionné en fonction de l'erreur d'alignement déterminée en mesurant la torsion de la patte lorsque le membre antérieur est maintenu en position étendue et que le poignet est en hyperextension.

5. Plaque (100) selon l'une des revendications 1 à 4,
dans laquelle la plaque (100) est adaptée pour être fixée au côté latéral-caudal du cubitus.

6. Plaque (100) selon l'une des revendications 1 à 5,
adaptée pour ajuster l'angle du compartiment médial d'un cubitus chez un chien.
